# EUROPEAN PATENT SPECIFICATION

(11) **EP 3 603 580 B1**
(45) Date of publication and mention of the grant of the patent: **06.12.2023**
(21) Application number: 19189225.6
(22) Date of filing: 30.07.2019
(51) Int. Cl.: A61F 2/44, A61F 2/30

(54) **INTERVERTEBRAL SPINAL IMPLANTS**
ZWISCHENWIRBELSÄULENIMPLANTATE
IMPLANTS SPINAUX INTERVERTÉBRAUX

(30) Priority: 02.08.2018 US 201816053300
(43) Date of publication of application: 05.02.2020
(73) Proprietor: Globus Medical, Inc., Audubon, PA 19403 (US)
(72) Inventor: CRYDER, Joel, WARRINGTON, PA 18976 (US); FEIGENBAUM, David, PHILADELPHIA, PA 19103 (US); KOPP, Hillary, Virginia Beach, VA 23451 (US); WEIMAN, Mark, DOWNINGTOWN, PA 19335 (US); MICCIO, Mark, LYNBROOK, NY 11563 (US)
(74) Representative: Morabito, Sara

(56) References cited:
- WO-A1-2017/106780
- US-A- 3 867 728
- US-A1- 2003 045 939
- US-A1- 2008 021 556
- US-A1- 2015 094 813

## Description

### CROSS-REFERENCE TO RELATED APPLICATION

The present application is a continuation-in-part of U.S. Patent Application No. 15/815,091, filed on November 16, 2017, which is continuation-in-part of U.S. Application Serial No. 15/189,188, filed on June 22, 2016, which is a continuation-in-part of U.S. 15/014,189, filed February 3, 2016.

The present application is also a continuation-in-part of U.S. Patent Application No. 15/973,609 filed on May 8, 2018.

### FIELD

The present application relates to intervertebral implants configured to promote intervertebral fusion and associated methods thereof (methods are not claimed).

### BACKGROUND

The goal of spine surgery may be for fusion of the two vertebrae adjacent to the targeted disc level, which may be accomplished through an interbody cage procedure, for example. The endplates of the implant come into contact with the patient's vertebral endplates, and the structure of the implant's endplates may be used to promote fusion between the implant and the vertebral endplates. Patent document WO2017106780 discloses such an implant.

Fusion may also be enhanced by packing the implant with a graft material. Prepacking an implant with graft material can result in some graft material falling out during the insertion procedure as well as missing out on available void space, such as for expandable implants. After expansion, some expandable implants will have an increase of volume that could be used as a graft window; however, this volume cannot be pre-packed because of the expansion of space after implantation.

Post-packing the implant may also be used to fill the implant with graft material. For example, the implant may be backfilled through the inserter or post-packed with another instrument. A complication that may arise when post-packing without the inserter is that it can be difficult to engage the implant once the inserter is removed. Therefore, it may be beneficial to provide a system that allows for better post-packing of the implant.

### SUMMARY

To meet this and other needs, endplates having geometries designed for enhanced bone fusion, intervertebral implants, such as expandable implants, utilizing such endplates, and methods of increasing bone graft packing are provided. The endplate geometries and improved packing methods may promote and enhance bone growth and fusion.

According to one embodiment, an implant, such as an expandable implant, includes a superior endplate and an inferior endplate. Each of the endplates may include a first portion having a solid structure and a second portion having a porosity. The first portion comprises a plurality of transverse struts having voids located between the struts and the second portion comprises a lattice structure formed in the voids of the solid structure.

In another embodiment, an intervertebral implant includes a superior endplate and an inferior endplate expandably connected to the superior endplate. Each of the superior endplate and the inferior endplate includes a rigid structure having a plurality of voids formed therein, wherein a lattice structure is formed in at least some of the plurality of voids.

In still another embodiment, an intervertebral implant includes an endplate comprising a first portion defining a central opening extending therethrough and a plurality of voids formed therein. A second portion having a lattice structure defined by a plurality of pores is located around the generally central opening and in at least some off the plurality of voids.

In an alternative embodiment, a bone funnel tube assembly can be used to inject graft material into the implant after the implant has been implanted into a patient. The bone funnel tube includes a hollow inserter that is releasably attached to the implant and is used to insert the implant between two adjacent vertebral discs. A funnel tube (not claimed) is inserted into and can be releasably attached to the inserter to prevent the funnel tube from inadvertently dislodging. A funnel is attached to a proximal end of the funnel tube to assist in inserting the graft material into the funnel tube. A graft pusher can be inserted into the funnel tube through the funnel to push the graft material through the funnel tube.

Also provided are methods (not claimed) for installing, expanding, and backfilling the implants, and kits (not claimed) including implants and components for the same.

### BRIEF DESCRIPTION OF THE DRAWINGS

Other aspects, features, and advantages of the present device will become more fully apparent from the following detailed description, the appended claims, and the accompanying drawings in which like reference numerals identify similar or identical elements.
FIG. 1 is a perspective view of an implant assembly in a compressed or collapsed condition according to an exemplary embodiment;
FIG. 2 is a perspective view of the implant assembly of FIG. 1 in an expanded condition, with the lattice structure of FIG. 1 removed to show the solid structure;
FIG. 3 is a top plan view of the implant assembly of FIG. 2 with the lattice structure of FIG. 1 removed to show the solid structure;
FIG. 4 is a top plan view of the lattice structure of the endplate shown in FIG. 1;
FIG. 5 is a perspective view of a superior endplate of the implant assembly of FIG. 1;
FIG. 6 is a side elevational view of the implant assembly of FIG. 2, in a compressed or collapsed condition;
FIG. 7 is a sectional view of the implant assembly of FIG 2;
FIG. 8 is an exploded perspective view of a bone funnel tube assembly according to an exemplary embodiment;
FIG. 9 is a side elevational view of the bone funnel tube assembly of FIG. 8;
FIG. 10 is an exploded perspective view of a bone funnel tube assembly according to an alternative exemplary embodiment;
FIG. 11 is an enlarged side elevational view of a pushbutton mechanism attached to an inserter of the assembly of FIG. 10;
FIG. 12 is a sectional view of the push-button assembly of FIG. 11, taken along lines 12-12 of FIG. 11;
FIG. 13 is a side elevational view, in section, of the push-button assembly of FIG. 11, with a bone funnel tube fully inserted therein;
FIG. 14 is a side elevational view, in section, of the push-button assembly of FIG. 11, with a bone funnel tube only partially inserted therein;
FIG. 15 is a side elevational view of a proximal end of the bone funnel tube of the assembly of FIG. 10;
FIG. 16 is an exploded perspective view of a bone funnel tube assembly according to another alternative exemplary embodiment;
FIG. 17 is a side elevational view of a proximal end of the bone funnel tube of the assembly of FIG. 16;
FIG. 18 is a side elevational view, in section, of a threaded connection of the assembly of FIG. 16;
FIG. 19 is an exploded perspective view of a bone funnel tube assembly according to yet another alternative exemplary embodiment;
FIG. 20 is a side elevational view, in section, of a spring connection of the assembly of FIG. 19; and
FIG. 21 is a graft pusher used with any of the assemblies of FIGS. 8-20.

### DETAILED DESCRIPTION

In the drawings, like numerals indicate like elements throughout. Certain terminology is used herein for convenience only and is not to be taken as a limitation on the present device. The terminology includes the words specifically mentioned, derivatives thereof and words of similar import. As used herein, the term "proximal" is defined as a direction closer to a clinician inserting the bone funnel tube and the term "distal" is defined as a direction farther from the clinician inserting the bone funnel tube.

The embodiments illustrated below are not intended to be exhaustive or to limit the device to the precise form disclosed. These embodiments are chosen and described to best explain the principle of the device and its application and practical use and to enable others skilled in the art to best utilize the device.

Reference herein to "one embodiment" or "an embodiment" means that a particular feature, structure, or characteristic described in connection with the embodiment can be included in at least one embodiment of the device. The appearances of the phrase "in one embodiment" in various places in the specification are not necessarily all referring to the same embodiment, nor are separate or alternative embodiments necessarily mutually exclusive of other embodiments.

As used in this application, the word "exemplary" is used herein to mean serving as an example, instance, or illustration. Any aspect or design described herein as "exemplary" is not necessarily to be construed as preferred or advantageous over other aspects or designs. Rather, use of the word exemplary is intended to present concepts in a concrete fashion.

Additionally, the term "or" is intended to mean an inclusive "or" rather than an exclusive "or". That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. In addition, the articles "a" and "an" as used in this application and the appended claims should generally be construed to mean "one or more" unless specified otherwise or clear from context to be directed to a singular form.

Unless explicitly stated otherwise, each numerical value and range should be interpreted as being approximate as if the word "about" or "approximately" preceded the value of the value or range.

The use of figure numbers and/or figure reference labels in the claims is intended to identify one or more possible embodiments of the claimed subject matter in order to facilitate the interpretation of the claims. Such use is not to be construed as necessarily limiting the scope of those claims to the embodiments shown in the corresponding figures.

It should be understood that the steps of any exemplary (not claimed) methods set forth herein are not necessarily required to be performed in the order described, and the order of the steps of such methods should be understood to be merely exemplary.

Also for purposes of this description, the terms "couple," "coupling," "coupled," "connect," "connecting," or "connected" refer to any manner known in the art or later developed of joining or connecting two or more elements directly or indirectly to one another, and the interposition of one or more additional elements is contemplated, although not required.

According to one embodiment, an implant, such as an expandable implant, includes at least a portion of the device having a solid structure and another portion of the device having a lattice structure with a porosity defined by the lattice network. In particular, it may be desirable for the endplates to have a composite structure with a portion being solid and a portion being porous. In an exemplary embodiment, the implant device or components thereof (e.g., the endplates) may be 3D printed to obtain the composite structure (solid structure combined with porous structure). The parts may be 3D printed with materials, such as biocompatible materials, including metals, polymers, ceramics or combinations thereof. Biocompatible metals may include titanium, titanium alloys, cobalt-chrome, stainless steel, or the like and biocompatible plastics, such as PEEK may be suitable. In an exemplary embodiment, the material for both the solid structure and the lattice structure can be Ti-6Al-4V extra low interstitials (TAV ELI) according to ASTM standard F3001, although those skilled in the art will recognize that other materials can be used. It is also envisioned that the solid structure material could be different from the lattice structure material. It may be desirable, however, that the lattice material be porous or more porous relative to the solid structure material (e.g., non-porous). It is also contemplated that one or more components of the device (e.g., the inner workings of the implant) may be machined or otherwise produced according to standard techniques and are not necessarily 3D printed components.

Various forms of additive manufacturing, or 3D printing, have been developed which allow structures to be formed layer by layer. One illustrative 3D printing technology is Direct Metal Laser Sintering (DMLS) or Selective Laser Melting (SLM) where parts are built using a laser to selectively sinter (heat and fuse) a powdered metal material into layers. The process begins once a 3D CAD file is mathematically sliced into multiple 2D cross sections and uploaded into the system. After the first layer is produced, the build platform is lowered, another powder layer is spread across the plate, and the laser sinters the second layer. This process is repeated until the part is complete. Layer-by-layer manufacturing allows for the direct fabrication of complex parts that would be cost-prohibitive, and often impossible, to produce through traditional manufacturing processes. The powder layer thickness used during the fabrication of the spacers may be as thin at 30µm. The resolution of the laser may be as fine as 70µm. Although it is envisioned that any suitable thickness or laser resolution may be used or selected.

The disclosure is not limited to DMLS, but various 3D printing methods may be utilized. For example, VAT Photopolymerization utilizes a vat of liquid photopolymer resin which is cured through selective exposure to light (via a laser or projector) which then initiates polymerization and converts the exposed areas to a solid part. As another example, Powder Bed Fusion, of which DMLS is a subcategory, utilizes powdered materials which are selectively consolidated by melting it together using a heat source such as a laser or electron beam. The powder surrounding the consolidated part acts as support material for overhanging features. As yet another example, in Binder Jetting Liquid bonding agents are selectively applied onto thin layers of powdered material to build up parts layer by layer. The binders include organic and inorganic materials. Metal or ceramic powdered parts are typically fired in a furnace after they are printed. Material Jetting is another example of a 3D printing process which may be utilized wherein droplets of material are deposited layer by layer to make parts. Common varieties include jetting a photocurable resin and curing it with UV light, as well as jetting thermally molten materials that then solidify in ambient temperatures. As another example, in Sheet Lamination sheets of material are stacked and laminated together to form an object. The lamination method can be adhesives or chemical (paper/plastics), ultrasonic welding, or brazing (metals). Unneeded regions are cut out layer by layer and removed after the object is built. Another example of a 3D printing process that may be utilized is Material Extrusion wherein material is extruded through a nozzle or orifice in tracks or beads, which are then combined into multi-layer models. Common varieties include heated thermoplastic extrusion and syringe dispensing. Yet another example is Directed Energy Deposition wherein powder or wire is fed into a melt pool which has been generated on the surface of the part where it adheres to the underlying part or layers by using an energy source such as a laser or electron beam.

The implants of the disclosure may be manufactured from any of these or other additive manufacturing processes currently known or later developed. The implants may also be manufactured utilizing a combination of additive manufacturing processes and other manufacturing processes, for example, laser etching. Additionally, the implants may be further processed during and/or after manufacture utilizing various techniques, for example, abrasion, machining, polishing, or chemical treatment.

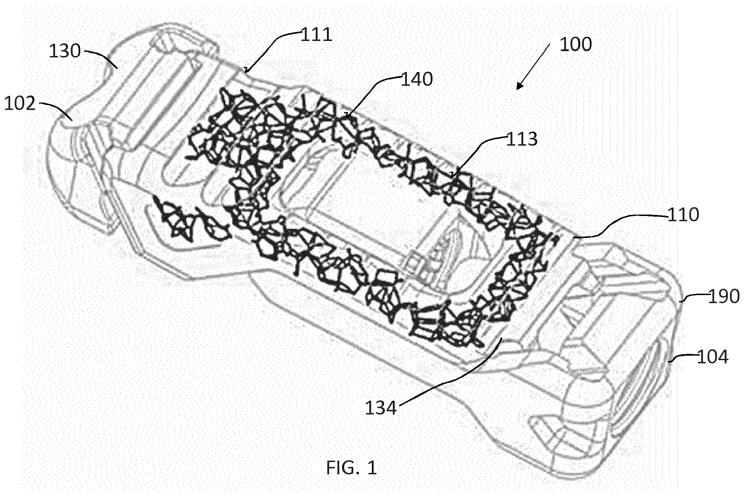

Referring to FIGS. 1-7, an intervertebral implant 100 ("implant 100") according to a first exemplary embodiment is shown. Implant 100 includes a superior endplate 110 and an inferior endplate 150 that is adjacent to and expandably connected to the superior endplate 110 such that the superior endplate 110 is expandable away from the inferior endplate 150. The implant 100 extends from a first, anterior end 102 to a second, posterior end 104, such that the first end 102 is configured to be inserted into the intervertebral space first.

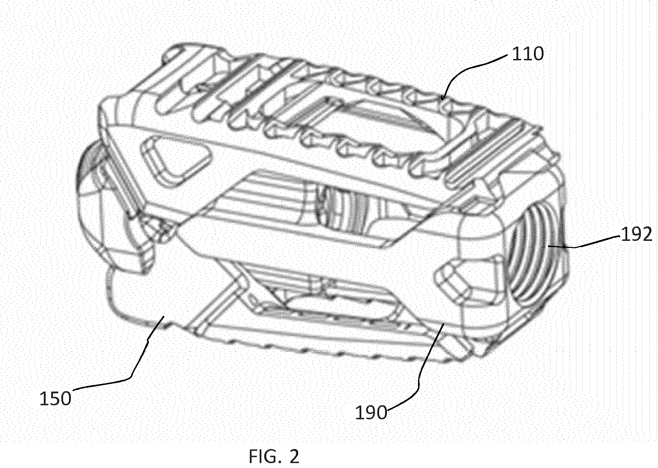

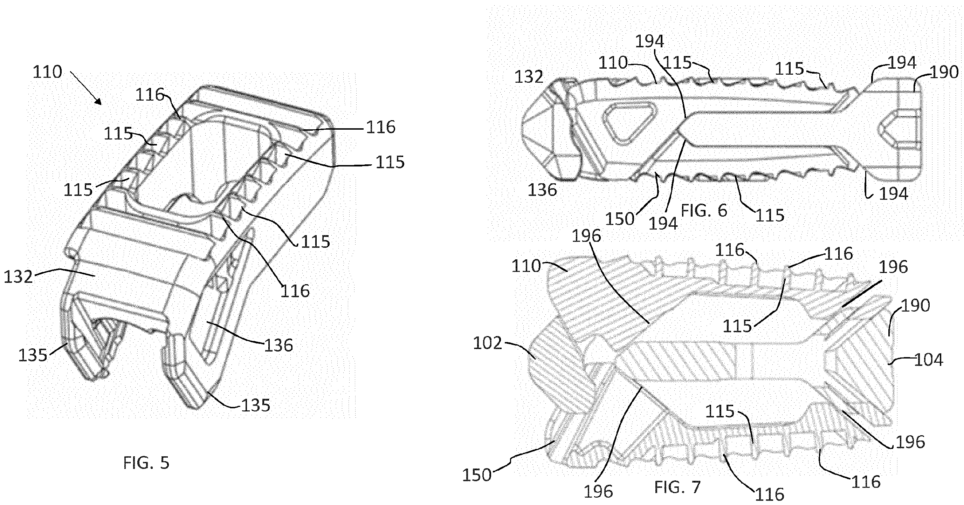

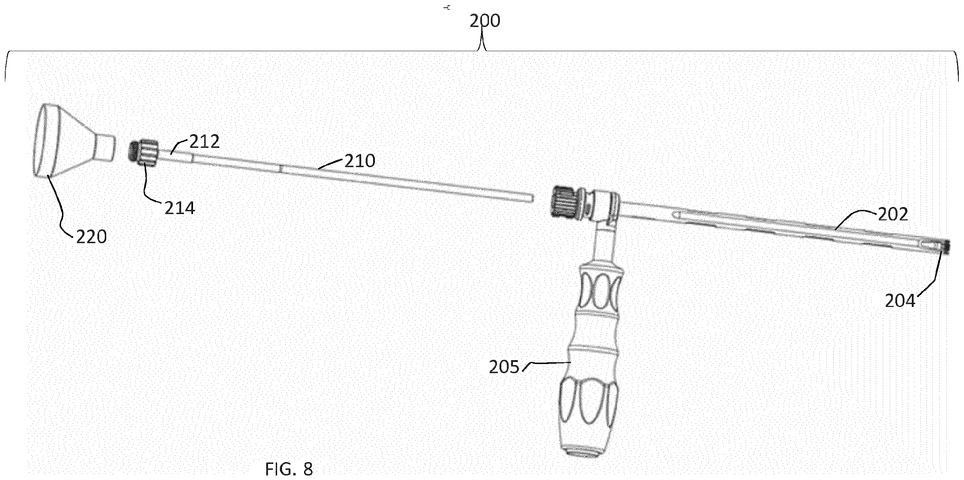

In an exemplary embodiment, shown in FIG. 2, an expander 190 is adjustably located between the superior endplate 110 and the inferior endplate 150. The expander 190 is used to adjust the vertical height of the superior endplate 110 with respect to the inferior endplate 150. For example, the expander 190 may include one or more ramped surfaces 194 configured to engage one or more ramped surfaces 196 on the superior and inferior endplates 110, 150, respectively. As the expander 190 move in a first direction, the ramped surfaces 194, 196 engage with one another to allow the superior and inferior endplates 110, 150 to move away from one another and into an expanded position. Conversely, when the expander 190 is moved in a second direction, opposite the first direction, the superior and inferior endplates 110, 150 move toward one another to a contracted position (or partially expanded position). As shown in FIG. 7, when expanded, the upper and lower surfaces of the superior and inferior endplates 110, 150 are not parallel to one another. The anterior end 102 is expanded to a height greater than the posterior end 104 to correct lordosis or the like. It is envisioned, however, that the endplates 110, 150 may expand in parallel or another configuration. The expander 190 also provides a connecting point at a threaded connection 192 for an inserter (shown in FIG. 8) to releasably connect to the implant 100 for insertion. The mechanics of such expandable devices is further described in detail in U.S. Patent Application No. 15/815,091, filed on November 16, 2017, now U.S. Publication No. 2018/0116817.

The expanded implant 100 provides for disc height restoration and ensures maximum contact with the vertebral endplates (not shown) of the patient. Those skilled in the art, however, will recognize that, for purposes of this disclosure, implant 100 can omit the expander 190 and merely include a superior surface (superior endplate 110) and an inferior surface (inferior endplate 150) with an integral body therebetween, for example, as a traditional interbody fusion spacer or scaffold.

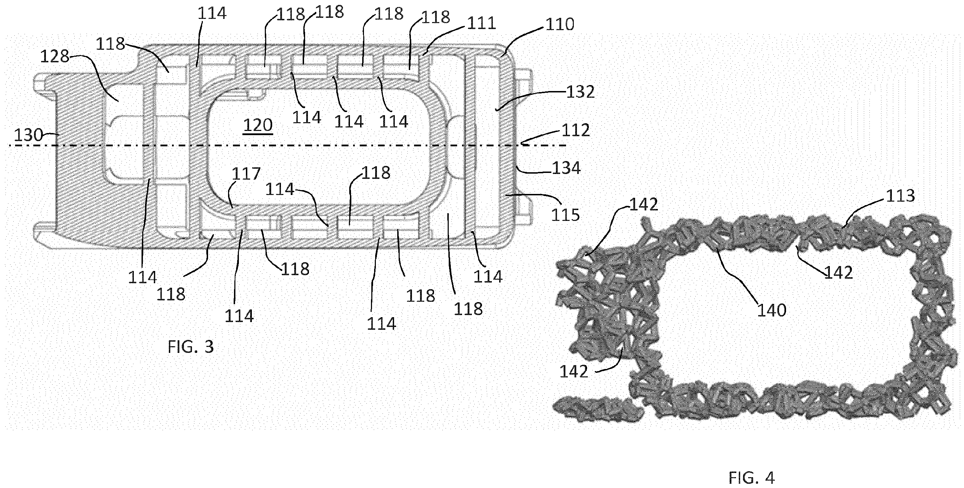

Referring to FIG. 1, the implant 100 combines solid and porous portions to enhance structural stability and bone growth. The endplates 110, 150 are constructed from a solid portion 111, best seen in FIG. 3, and a porous portion 113 best seen in FIG. 4 in isolation. The solid portion 111 may be comprised a plurality of walls, bands, or struts 114. The solid portion 111 may be provided around a perimeter of the endplate 110, 150 thereby forming a continuous outer wall 115. The solid portion 111 may also form a continuous wall 117 around the central opening 120. A plurality of inner walls or struts 114 may be provided through central portions of the endplate 110, 150 to provide adequate structural integrity to the endplates 110, 150. A lattice structure 140 containing the porous portions or pores 142 may be formed over at least a portion of the endplates 110, 150. Although shown with reference to the superior endplate 110, the lattice structure 140 may also be formed over at least a portion of the inferior endplate 150 (in the same or a different manner than endplate 110). For purposes of simplification, superior endplate 110 is shown and described but it is understood that the same features are provided for the inferior endplate 150. It is also envisioned that the location and volumes of solid portion 111 and porous portion 113 can be varied or changed along the endplates 110, 150 or any component of the implant 100 to enhance fusion and stability.

The solid portions 111 and/or the porous portions 113 may be constructed, for example, using a 3D printing process. The lattice structure 140 forms a geometry that includes a plurality of microscopic struts or micro-lattice structure oriented in a trabecular fashion, for example, to create a micro-porosity that has the potential to promote bone fusion.

The porosity of the lattice structure 140 may have a randomized pattern of open pores 142 or a repeating pattern of open pores 142. The lattice structure 140 may have a suitable porosity (open volume). For example, the porosity of the structure 140 may be greater than 50% open, greater than 60% open, greater than 70% open, or approximately 70% open, or approximately 75% open. The porosity of the structure 140 may feature interconnected pores or open pores. The porosity of the structure 140 may have pores, for example, ranging from approximately 100 µm -2mm, approximately 100 µm -1mm, approximately 200-900 µm, or approximately 300-800µm in diameter. The pore size may have an average pore size of about 300-800 µm, about 400-700 µm, or about 500-600 µm. The pore size distribution may be unimodal or bi-modal. Although spherical or partially-spherical pores or nodes are exemplified in forming the porous structure, it is envisioned that other suitable pore shapes and configurations may be used, for example, repeating or random patterns of cylinders, cubes, cones, pyramids, polyhedrons, or the like.

The combination of the porous and the solid material 111, 113 allows for a high strength to porosity ratio for implant 100. The advantage of this combination of solid and porous material 111, 113 allows for an increase in bone fusion due to the porosity characteristics of the lattice structure 140. The combination of porous and solid material 111, 113 allows for an increase in fusion while still maintaining the structural integrity required to support the necessary load that the implant 100 will be subjected to in the patient.

Referring to FIGS. 3 and 5, a portion of the superior endplate 110 is a rigid structure 111. The rigid structure 111 may be defined by solid walls and struts 114, 115, 117. The solid walls and struts 114, 115, 117 may provide a plurality of gaps or openings 118 that are partially or completely filled with the porous structure 113. The endplate 110 extends along a central longitudinal axis 112 and includes a plurality of inner struts 114 located across and in a first direction generally transverse to the central longitudinal axis 112. The plurality of inner struts 114 within the center of the implant may extend between an outer sidewall 115 extending around the outer perimeter of the implant and a sidewall 117 extending around the central opening 120. The inner struts 114 may be arranged generally perpendicularly to the lateral side walls and generally in parallel to the front and rear sidewalls at the anterior and posterior ends 102, 104, respectively, of the implant 100. The inner struts 114 may also be spaced apart such that the gaps 118 between adjacent struts 114 have a width at least equal to, but preferably, larger than a width of an adjacent strut 114. The inner struts 114 may also be uniformly distributed such that the spacing 118 between struts 114 is equal.

Inner struts 114 may include a plurality of teeth 115, if desired, at a superior end thereof that extend transverse to the central longitudinal axis 112 and enable the implant 100 to better grip the vertebral endplates. A side view of the collapsed implant 100 can be seen in FIG. 6 with the teeth 115 protruding out of both of the superior endplate 110 and the inferior endplate 150). Additionally, solid material ribs 116 are located along the apex of the teeth 115 to increase the strength and durability of the teeth 115 at the location of initial contact with the vertebral endplates. These solid ribs 116 can be seen in the cross sectional view of the expanded implant 100 in FIG. 7. A cross sectional view of the solid structure of endplates 110, 150, and expander 190 can be seen in FIG. 7. The solid material of these components is strategically located to provide structural integrity in areas of higher stress.

Referring back to FIG. 3, the solid portion 111 of the endplate 110 has a plurality of large voids or gaps 118 located between adjacent struts 114. At least some of the voids 118 are located along the central longitudinal axis 112. The struts 114 may be provided such that they extend from one sidewall of the implant to the other. Thus, the larger voids 118 may extend from sidewall to sidewall whereas smaller voids 118 may be formed between the sidewall of the central opening 120 and the outer sidewalls of the device.

The lattice structure 140 is formed in at least some of the plurality of voids 118, thereby connecting struts 114 to one another. In some embodiments, at least some of the plurality of voids can be devoid of the lattice structure 140 and may remain open for graft material, for example. As shown, an elongate and enlarged central opening 120 may extend through the superior endplate 110 along the central longitudinal axis 112 and is devoid of any lattice structure.

While a significant portion of each endplate 110, 150 is a mixture of solid and porous material, there are specific sections of each endplate 110, 150 that are porous entirely through from top to bottom, such as a void 128 at a tapered front portion 130, and a void 132 at a rear portion 134 of each endplate 110, 150, as well as in between the teeth 115.

The geometry mentioned above allows for porous material to be placed throughout a thickness of the endplate 150 while still maintaining its structural integrity. Additionally, the tapered front portion 130 of the endplate 150, shown in FIG. 1, can be composed of the solid material. The tapered front portion 130 provides for a smoother surface during insertion of the implant 100 between the vertebral members. During the insertion procedure, as the implant 100 is being impacted into the vertebral space between the vertebral members, the front portion 130 of the endplate 150 engages the vertebral members first. Rather than having a rough, porous surface contacting the vertebral members and risking the possibility of damaging the adjacent vertebrae, the smooth surface of the solid front portion 130 reduces the potential of damage to the vertebral endplates.

The front portion 130 tapers toward the inferior endplate 150, wherein the front portion is devoid of the porous material. The front portion 130 includes the tapered front portion 130 extending generally transverse to the plurality of struts 114. Referring to FIG. 5, the superior endplate 110 also includes a side wall 135 extending inferiorly therefrom on either side of the longitudinal axis 112. Each side wall 135 has a void 136 formed therein and the second material in the form of the lattice structure is provided in the void 136. The porous material 113 in the void 136 can be utilized as a radiographic marker when capturing fluoroscopic images. Because there may be porous material 113 at this location, the porous material 113 will appear lighter than the solid material 111 surrounding it, making it easily identifiable on fluoroscopic images.

After implant 100 (or any other implant) is inserted between adjacent vertebrae, it may be desirable to add graft material to the implant 100. It is particularly desirous to add graft material after insertion if the implant is expandable (such as implant 100), due to the fact that the expansion of the implant is performed during the implantation process and can increase the volume available for graft filling.

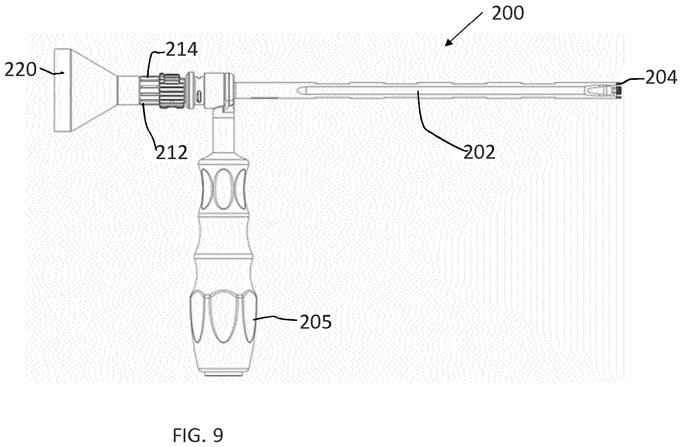

To insert graft material into implant 100, an exemplary embodiment of a bone funnel tube assembly 200, shown in FIGS. 8 and 9, is provided. A hollow inserter 202 has a distal end 204 that is threaded to engage the threads 192 on the expander 190. The inserter 202 is connected to a handle 205 that a clinician grasps to manipulate the inserter 202 and the implant 100 during insertion of the implant 100.

Bone funnel tube assembly 200 has a funnel tube 210 that is inserted into the inserter 202 to direct graft material through the inserter 202 and into voids in the implant 100. A funnel 220 is attached to a proximal end 212 of the funnel tube 210. The proximal end 212 of the funnel tube 210 includes a longitudinally ribbed knob 214 that allows a clinician to readily grip the funnel tube 210 to insert the funnel tube 210 into the inserter 202.

In an exemplary embodiment, the funnel 220 can be fixedly connected to funnel tube 210. In an alternative embodiment, the funnel 220 can be releasably connected to the funnel tube 210, such as by a threaded connection. In this exemplary embodiment, the funnel tube 210 is merely slidingly inserted into the inserter 202, without any connection between the inserter 202 and the funnel tube 210.

It can be advantageous to restrict the motion of the bone funnel relative to the inserter and prevent the funnel tube from inadvertently disengaging from the inserter. Alternative embodiments that restrict motion of the bone funnel with respect to the inserted are described below with reference to bone funnel assemblies 300, 400, 500. In all of the described embodiments, the bone funnel can axially translate down the shaft of the inserter until the bone funnel bottoms out at the proximal face of the inserter.

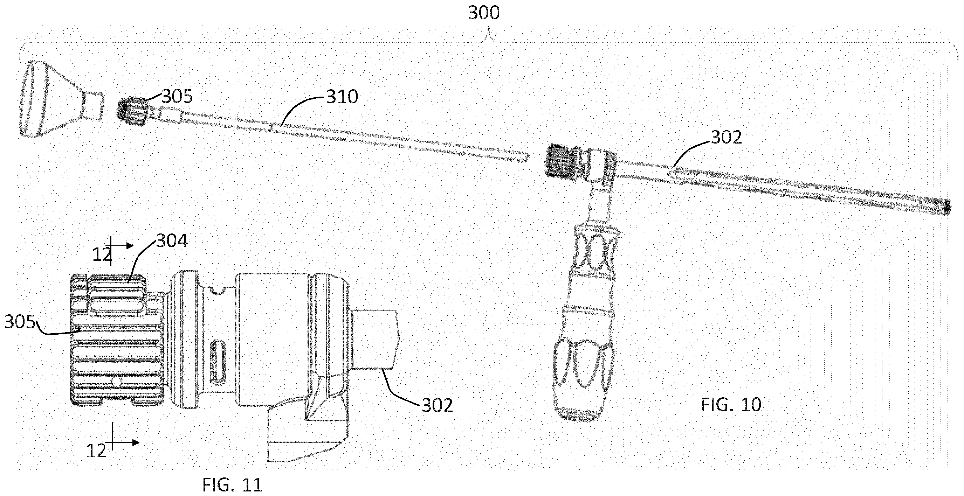

A bone funnel tube assembly 300 according to an alternative exemplary embodiment is shown in FIGS. 9-15. An inserter 302 includes a pushbutton spring locking mechanism 304 in a knurled handle 305 that is located at the proximal end of the inserter 302. Mechanism 304 is used to restrict axial translation of the bone funnel tube 310 with respect to the inserter 302 once the bone funnel tube 310 bottoms out against the inserter 302.

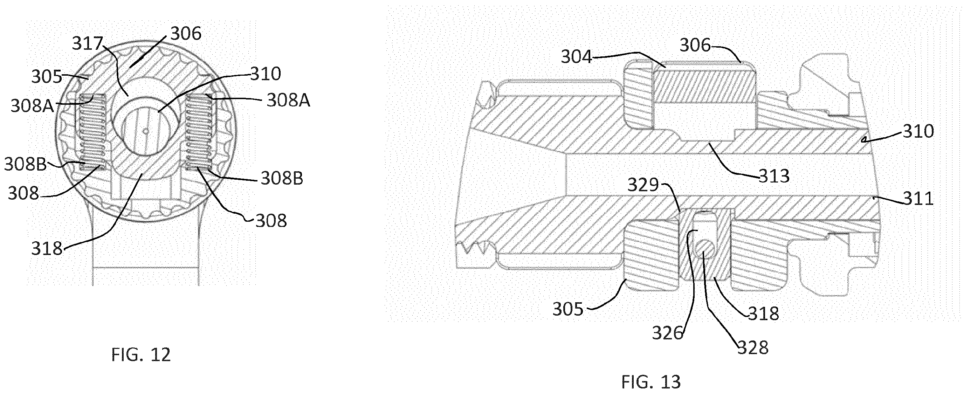

Mechanism 304 includes a pushbutton 306 that is biased away from the bone funnel tube 310 by biasing members 308, shown in FIG. 12. In an exemplary embodiment, the biasing members 308 are helical springs, although those skilled in the art will recognize that other types of biasing members can be used. The biasing members 308 are disposed diametrically opposite each other alongside the bone funnel tube 210.

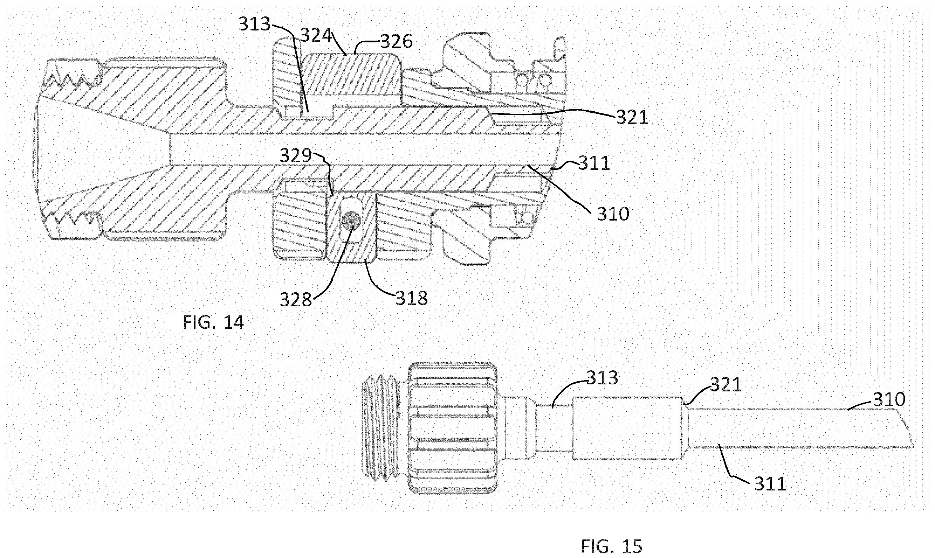

A first end 308A of biasing members 308 engages an interior surface of the pushbutton 306, while a second end 308B of the biasing members 308 engages an interior face 316 of the handle 305. The pushbutton 306 includes a tang 318 that wraps around the bone funnel tube 210 and extends into a slot 319 in the handle 305. Referring to FIG. 13, the tang 318 includes a transverse oblong slot 326 that receives a retaining pin 328. Pushbutton 306 also includes a through-opening 317 such that the bone funnel tube 310 can extend therethrough. The retaining pin 328 is used to retain the tang 318 in the slot 326 to retain the pushbutton 306 in the handle 305. A proximal end 329 of the tang 318, radially inwardly on the tang 318, is beveled to allow a tapered portion 321 of the funnel tube 310 (shown in FIGS. 14 and 15) to engage the tang 318 and bias the tang from the position shown in FIG. 13 to the position shown in FIG. 14 as the funnel tube 310 is fully advanced into the inserter 302. The geometry of the tapered portion 321 forces the tang 318 to translate radially away from the funnel tube 310 as the funnel tube 310 is advanced distally within the inserter 302.

The funnel tube 310 includes a shaft 311 having a radial notch 313 that aligns with the tang 318 when the funnel tube 310 is bottomed out against the proximal end of the inserter 302. The notch 313 in the funnel tube shaft 311 allows the pushbutton 306 to spring back and return to its original location (shown in FIG. 13) when released. While the pushbutton 306 sits in the notch 313, the bone funnel 310 is restricted from backing out of the inserter 302 on its own.

Pressing on the top of the pushbutton 306 radially inwardly moves the tang 318 radially outwardly and out of the notch 313, allowing for the removal of the bone funnel 310 proximally from the inserter 302.

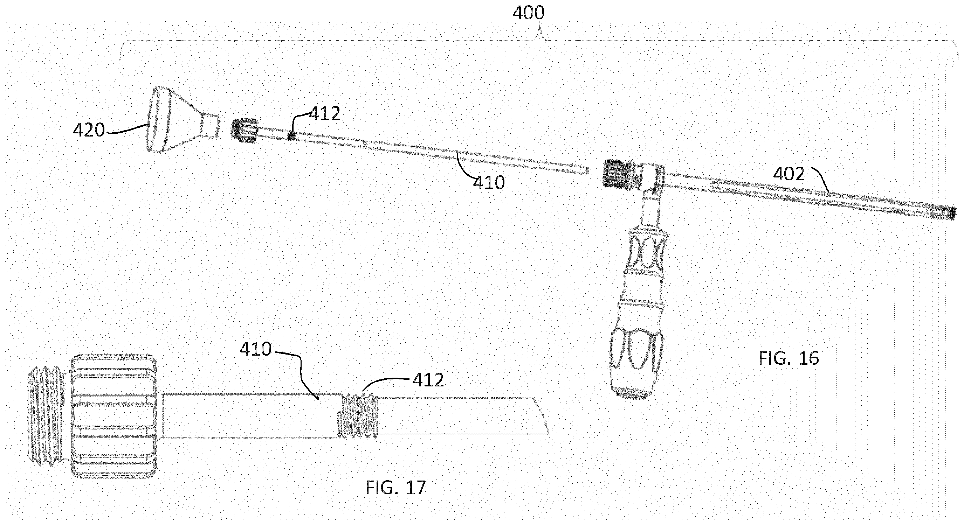

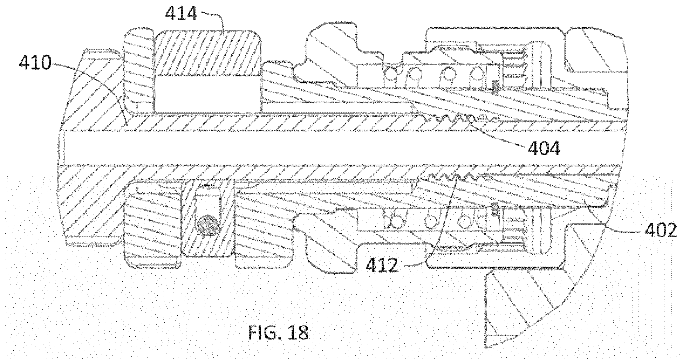

An alternative embodiment of a bone funnel tube assembly 400 is shown in FIGS. 16-18. A funnel tube 410 includes an exteriorly right-hand threaded portion 412, distal of the funnel 420, that mates with internal right-hand threads 404 on a hollow inserter 402. The threaded portion 412 and the internal threads 404 are located such that, when the threaded portion 412 engages the internal threads 404, the knob 414 bottoms out on the proximal end of the inserter 402, releasably securing the funnel tube 410 to the inserter 402.

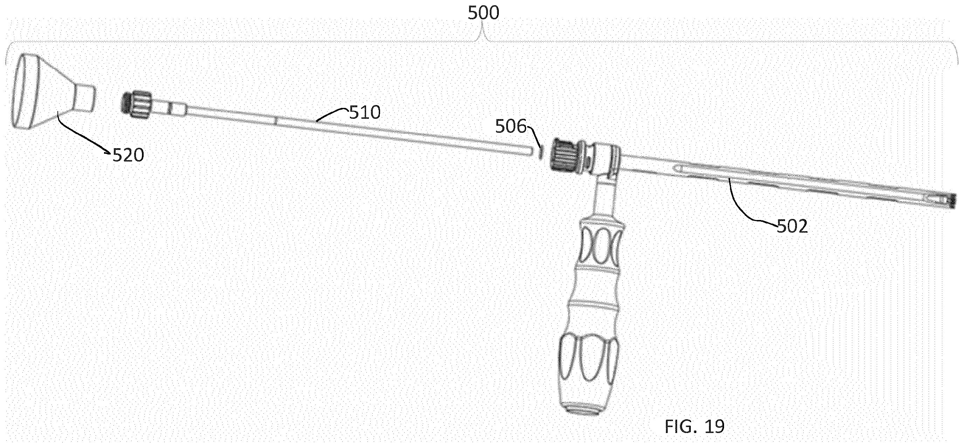

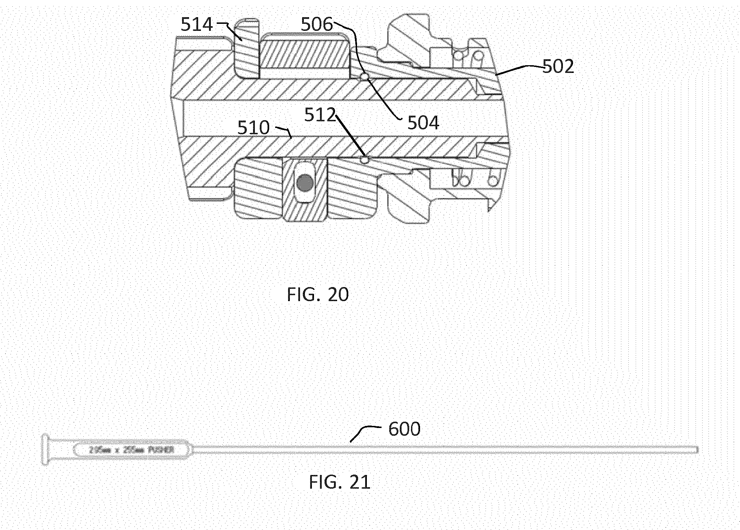

Another alternative embodiment of a bone funnel tube assembly 500 is shown in FIGS. 19 and 20. An inserter 502 includes an internal radially extending groove 504 located at a predetermined length along the inserter 502. A circular spring 506, such as, for example, a Bal Spring^{®} canted coil spring, is inserted into the groove 504.

A funnel tube 510 includes a radially extending groove 512 located at a predetermined length along the tube 510, distal from funnel 520, such that, when the funnel tube 510 is fully inserted into the inserter 502 and the knob 514 of the funnel tube 510 bottoms out on proximal end of the inserter 502, the grooves 504 and 512 line up with each other, allowing the spring 506 to expand into the groove 512 so that the spring 506 engages both the groove 504 and the groove 512, thereby securing the funnel tube 510 in the inserter 502. An effort on the part of the clinician is required to separate the bone funnel tube 510 from the inserter 502.

For any of assembly 200, 300, 400, 500, a graft pusher 600, shown in FIG. 21, can be inserted into the funnel tube 210, 310, 410, 510 through the funnel 220 to push the graft material through the funnel tube 210, 310, 410, 510 and into the implant, such as implant 100.

The implant may utilize endplates 110, 150 having geometries designed for enhanced bone fusion and improved packing methods may further promote and enhance bone growth and fusion.

It will be further understood that various changes in the details, materials, and arrangements of the parts which have been described and illustrated in order to explain the nature of this device may be made by those skilled in the art without departing from the scope of the device as expressed in the following claims.

## Claims

1. An intervertebral implant (100) comprising:
a superior endplate (110) having a sidewall (135) having a void (136) formed therein; and
an inferior endplate (150) separated from the superior endplate (110),
wherein each of the superior endplate (110) and the inferior endplate (150) comprises a first portion (111) having a solid structure and a second portion (113) having a porosity, wherein the first portion (111) comprises a plurality of transverse struts (114) having voids (118) located between the struts (114) and wherein the second portion (113) comprises a lattice structure (140) formed in the voids (118), and
wherein the lattice structure (140) is disposed only in the side void (136) and in the voids (118) in each of the superior endplate (110) and the inferior endplate (150).

2. The intervertebral implant according to claim 1, wherein the implant 100 comprises a body portion having a plurality of ramps (194) configured to engage with corresponding ramps (196) on the superior (110) and inferior endplates (150) , thereby allowing for expansion of the superior (110) and inferior endplates (150) away from one another.

3. The intervertebral implant according to claim 1, wherein the superior endplate (110) comprises a side wall (115) extending inferiorly therefrom, the side wall (135) having a void (136), wherein the lattice structure (140) is provided in the void (136).

4. The intervertebral implant according to claim 1, wherein the superior endplate (110) extends along a central longitudinal axis (112), and wherein the superior endplate (110) comprises an elongate void (120) extending along the central longitudinal axis (112).

5. The intervertebral implant according to claim 4, wherein the plurality of transverse struts (114) extend generally transverse to the central longitudinal axis (112).

6. The intervertebral implant according to claim 4, wherein the voids (118, 136) are located along the central longitudinal axis (112).

7. The intervertebral implant according to claim 4, wherein the superior endplate (110) comprises a plurality of teeth (115) extending transverse to the central longitudinal axis (112).

8. The intervertebral implant according to claim 1, wherein the superior endplate (110) comprises a front portion (130) constructed from the solid structure.

9. The intervertebral implant according to claim 8, wherein the front portion (130) tapers toward the inferior endplate (150).

10. The intervertebral implant according to claim 8, wherein the front portion (130) is devoid of the lattice structure.

11. The intervertebral implant according to claim 1, further comprising an expander (190) adjustably located between the superior endplate (110) and the inferior endplate (150).

12. The intervertebral implant according to any one of the preceding claims wherein the superior endplate (110) and an inferior endplate (150) are expandably connected one to another.

13. The intervertebral implant according to any one of the preceding claims wherein the lattice structure (140) is so positioned to come into contact with the bone of the patient.

## Patentansprüche

1. Zwischenwirbelimplantat (100), umfassend:
- eine obere Endplatte (110) mit einer Seitenwand (135), in der ein Hohlraum (136) ausgebildet ist; und
- eine untere Endplatte (150), die von der oberen Endplatte (110) getrennt ist,
- wobei sowohl die obere Endplatte (110) als auch die untere Endplatte (150) einen ersten Abschnitt (111) mit einer festen Struktur und einen zweiten Abschnitt (113) mit einer Porosität aufweisen, wobei der erste Abschnitt (111) eine Mehrzahl von Querstreben (114) mit zwischen den Streben (114) angeordneten Hohlräumen (118) aufweist und wobei der zweite Abschnitt (113) eine Gitterstruktur (140) aufweist, die in den Hohlräumen (118) ausgebildet ist, und
- wobei die Gitterstruktur (140) nur im seitlichen Hohlraum (136) und in den Hohlräumen (118) in sowohl der oberen Endplatte (110) als auch der unteren Endplatte (150) angeordnet ist.

2. Zwischenwirbelimplantat nach Anspruch 1, wobei das Implantat (100) einen Körperabschnitt mit einer Mehrzahl von Rampen (194) aufweist, die eingerichtet sind, um mit entsprechenden Rampen (196) an der oberen (110) und unteren Endplatte (150) in Eingriff zu kommen, wodurch eine Ausdehnung der oberen (110) und unteren Endplatte (150) voneinander weg ermöglicht wird.

3. Zwischenwirbelimplantat nach Anspruch 1, wobei die obere Endplatte (110) eine Seitenwand (115) aufweist, die sich von ihr nach unten erstreckt, wobei die Seitenwand (135) einen Hohlraum (136) aufweist, wobei die Gitterstruktur (140) im Hohlraum (136) vorgesehen ist.

4. Zwischenwirbelimplantat nach Anspruch 1, wobei sich die obere Endplatte (110) entlang einer zentralen Längsachse (112) erstreckt, und wobei die obere Endplatte (110) einen länglichen Hohlraum (120) aufweist, der sich entlang der zentralen Längsachse (112) erstreckt.

5. Zwischenwirbelimplantat nach Anspruch 4, wobei sich die Mehrzahl von Querstreben (114) im Wesentlichen quer zur zentralen Längsachse (112) erstreckt.

6. Zwischenwirbelimplantat nach Anspruch 4, wobei die Hohlräume (118, 136) entlang der zentralen Längsachse (112) angeordnet sind.

7. Zwischenwirbelimplantat nach Anspruch 4, wobei die obere Endplatte (110) eine Mehrzahl von Zähnen (115) aufweist, die sich quer zur zentralen Längsachse (112) erstrecken.

8. Zwischenwirbelimplantat nach Anspruch 1, wobei die obere Endplatte (110) einen vorderen Abschnitt (130) aufweist, der aus der massiven Struktur aufgebaut ist.

9. Zwischenwirbelimplantat nach Anspruch 8, wobei sich der vordere Abschnitt (130) zur unteren Endplatte (150) verjüngt.

10. Zwischenwirbelimplantat nach Anspruch 8, wobei der vordere Abschnitt (130) frei von der Gitterstruktur ist.

11. Zwischenwirbelimplantat nach Anspruch 1, das ferner einen Expander (190) aufweist, der einstellbar zwischen der oberen Endplatte (110) und unteren Endplatte (150) angeordnet ist.

12. Zwischenwirbelimplantat nach einem der vorhergehenden Ansprüche, wobei die obere Endplatte (110) und eine untere Endplatte (150) expandierbar miteinander verbunden sind.

13. Zwischenwirbelimplantat nach einem der vorangehenden Ansprüche, wobei die Gitterstruktur (140) positioniert ist, um mit dem Knochen des Patienten in Kontakt zu kommen.

## Revendications

1. Implant intervertébral (100), comprenant :
une plaque d'extrémité supérieure (110) comportant une paroi latérale (135) comportant un vide (136) formé en son sein ; et
une plaque d'extrémité inférieure (150) séparée de la plaque d'extrémité supérieure (110),
dans lequel chacune de la plaque d'extrémité supérieure (110) et de la plaque d'extrémité inférieure (150) comprend une première partie (111) ayant une structure solide et une seconde partie (113) ayant une porosité, dans lequel la première partie (111) comprend une pluralité d'entretoises transversales (114) comportant des vides (118) situés entre les entretoises (114) et dans lequel la seconde partie (113) comprend une structure en treillis (140) formée dans les vides (118), et
dans lequel la structure en treillis (140) n'est disposée que dans le vide latéral (136) et dans les vides (118) de chacune de la plaque d'extrémité supérieure (110) et de la plaque d'extrémité inférieure (150).

2. Implant intervertébral selon la revendication 1, dans lequel l'implant (100) comprend une partie corps comportant une pluralité de plans inclinés (194) conçus pour coopérer avec des plans inclinés correspondants (196) des plaques d'extrémité supérieure (110) et inférieure (150), permettant ainsi un déploiement des plaques d'extrémité supérieure (110) et inférieure (150) à l'opposé l'une de l'autre.

3. Implant intervertébral selon la revendication 1, dans lequel la plaque d'extrémité supérieure (110) comprend une paroi latérale (115) s'étendant de manière inférieure à partir de cette dernière, la paroi latérale (135) comportant un vide (136), dans lequel la structure en treillis (140) est disposée dans le vide (136).

4. Implant intervertébral selon la revendication 1, dans lequel la plaque d'extrémité supérieure (110) s'étend le long d'un axe central longitudinal (112), et dans lequel la plaque d'extrémité supérieure (110) comprend un vide allongé (120) s'étendant le long de l'axe central longitudinal (112).

5. Implant intervertébral selon la revendication 4, dans lequel les entretoises de la pluralité d'entretoises transversales (114) s'étendent globalement transversalement à l'axe central longitudinal (112).

6. Implant intervertébral selon la revendication 4, dans lequel les vides (118, 136) sont situés le long de l'axe central longitudinal (112).

7. Implant intervertébral selon la revendication 4, dans lequel la plaque d'extrémité supérieure (110) comprend une pluralité de dents (115) s'étendant transversalement à l'axe central longitudinal (112).

8. Implant intervertébral selon la revendication 1, dans lequel la plaque d'extrémité supérieure (110) comprend une partie avant (130) construite à partir de la structure solide.

9. Implant intervertébral selon la revendication 8, dans lequel la partie avant (130) forme un biseau en direction de la plaque d'extrémité inférieure (150).

10. Implant intervertébral selon la revendication 8, dans lequel la partie avant (130) est dépourvue de la structure en treillis.

11. Implant intervertébral selon la revendication 1, comprenant en outre un dispositif de déploiement (190) situé de manière réglable entre la plaque d'extrémité supérieure (110) et la plaque d'extrémité inférieure (150).

12. Implant intervertébral selon l'une quelconque des revendications précédentes, dans lequel la plaque d'extrémité supérieure (110) et une plaque d'extrémité inférieure (150) sont reliées l'une à l'autre de manière à pouvoir être déployées.

13. Implant intervertébral selon l'une quelconque des revendications précédentes, dans lequel la structure en treillis (140) est positionnée de façon à venir en contact avec l'os du patient.
